(19) Europäisches Patentamt
European Patent Office
Office européen des brevets

(11) **EP 1 600 891 A1**

(12) **EUROPEAN PATENT APPLICATION**

(43) Date of publication:
**30.11.2005 Bulletin 2005/48**

(51) Int Cl.⁷: **G06T 7/00, A61B 8/14,**
**A61B 8/08, G01S 7/52**

(21) Application number: **05009137.0**

(22) Date of filing: **26.04.2005**

(84) Designated Contracting States:
**AT BE BG CH CY CZ DE DK EE ES FI FR GB GR
HU IE IS IT LI LT LU MC NL PL PT RO SE SI SK TR**
Designated Extension States:
**AL BA HR LV MK YU**

(30) Priority: **27.05.2004 JP 2004157491**

(71) Applicant: **Aloka Co. Ltd.**
**Mitaka-shi Tokyo, 181-8622 (JP)**

(72) Inventors:
• **Murashita, Masaru**
**Mitaka-shi, Tokyo, 181-8622 (JP)**
• **Hirota, Koji**
**Mitaka-shi, Tokyo, 181-8622 (JP)**

(74) Representative: **Heim, Hans-Karl et al**
**Weber & Heim**
**Patentanwälte**
**Irmgardstrasse 3**
**81479 München (DE)**

(54) **Ultrasonic diagnostic apparatus and image processing method**

(57)     A three-dimensional image data memory (16) stores three-dimensional image data including that of a imaging target. A digitization processor unit (18) applies a digitizing process to the three-dimensional image data using a first threshold value to extract data corresponding to the target. A user sets a two-dimensional region of interest surrounding a portion in which the extraction of data using the first threshold value is inaccurate and a 3D region-of-interest generator unit (42) generates a three-dimensional region of interest from the two-dimensional region of interest which is set. The digitization processor unit (18) applies a digitizing process using a second threshold value to the three-dimensional image data within the three-dimensional region of interest.

Fig. 1

## Description

BACKGROUND OF THE INVENTION

1. Field of the Invention

The present invention relates to an ultrasonic diagnostic apparatus and an image processing method, and in particular to a technique for extracting an image of a target from an ultrasonic image or the like.

2. Description of the Related Art

An ultrasonic diagnostic apparatus transmits and receives an ultrasound to and from a space including a target (such as a body organ, a cavity within an organ, a tumor, or the like) to obtain echo data and generates an ultrasonic image such as a cross-sectional image and a three-dimensional image based on the echo data. In general, an ultrasonic image includes images of areas other than the target. As such, techniques are known for extracting an image of only the target in order to improve precision of diagnosis or the like.

For example, a technique is known in which, in order to extract data of an organ which is a diagnosis target from an echo data space, a three-dimensional region is designated along an outline of the organ data within the echo data space and a display image is generated using the echo data within the three-dimensional region (Japanese Patent Laid-Open Publication No. 2004-33658, etc.). Another technique is also known in which an ultrasonic cross-sectional image is divided into a plurality of sub-regions, image processes such as digitization are applied for each sub-region, and outline information which represents an outline of a target is obtained (Japanese Patent Laid-Open Publication No. 2003-334194, etc.).

However, in order to accurately extract organ data from the three-dimensional region using the method disclosed in Japanese Patent Laid-Open Publication No. 2004-33658, it is necessary to accurately set the three-dimensional region along the outline of the organ based on the shape of the organ.

In the method disclosed in Japanese Patent Laid-Open Publication No. 2003-334194, because a plurality of sub-regions radially divided by a predetermined angle from the center of gravity are set, the sub-regions sometimes do not surround the region of interest. Depending on the target, the boundary may not be clear at a specific portion of the target, and there may be cases in which it is desired to apply a special image process regarding the specific portion.

SUMMARY OF THE INVENTION

The present invention advantageously provides a device for suitably separating tissue or the like in a region of interest.

According to one aspect of the present invention, there is provided an ultrasonic diagnostic apparatus comprising an image data generation unit which transmits and receives an ultrasound to and from a space including a target (target part, target portion, target area, etc.) to generate ultrasonic image data, a target extraction unit which applies a digitizing process to the ultrasonic image data using a first threshold value to extract data corresponding to the target, and a region-of-interest setting unit which sets a region of interest within the ultrasonic image data, wherein the target extraction unit applies a digitizing process, using a second threshold value, to the ultrasonic image data within the region of interest which is set. According to another aspect of the present invention, it is preferable that, in the ultrasonic diagnostic apparatus, the region-of-interest setting unit sets a region of interest surrounding a portion within the ultrasonic image data in which the extraction of data using the first threshold value is inaccurate.

In the above-described structure, the ultrasonic image data is, for example, two-dimensional cross-sectional image data or three-dimensional image data. The ultrasonic diagnostic apparatus having the structure as described above is suited to diagnosing a liver cyst. That is, a liver cyst is an example of a preferable target. The target is, however, obviously not limited to a liver cyst and may be any other target suitable for imaging such as, for example, a heart or another organ, a cavity in an organ, a tumor, or the like. When the target is a liver cyst, for example, debris (secretion or the like) may be present in the liver cyst and the boundary of the liver cyst is not clear at the portion of the ultrasonic image corresponding to the debris. In other words, the echo of the debris creates noise, making the boundary between the liver cyst and external tissues unclear. With the above-described structure, a region of interest is set to surround a portion in which extraction of data is inaccurate and the ultrasonic image data is binarized using a second threshold value in the set region of interest. That is, by setting the region of interest to, for example, a region surrounding the debris portion, it is possible to apply a specific digitizing process solely to the debris portion using the second threshold value. With this configuration, it is possible to accurately determine the boundary between the liver cyst and the external tissue, even in the debris portion, simply by suitably setting the second threshold value.

According to another aspect of the present invention, there is provided an ultrasonic diagnostic apparatus comprising a three-dimensional image data generation unit which transmits and receives an ultrasound to and from a space including a target to generate three-dimensional image data, a target extraction unit which applies a digitizing process to the three-dimensional image data using a first threshold value to extract data corresponding to the target, a cross-sectional image data generation unit which generates, from the three-dimensional image data, cross-sectional image data after the digitizing process using the first threshold value, and a three-dimensional region-of-interest setting unit which sets a three-dimensional region of interest within the three-dimensional image data based on a two-dimensional region of interest which is set within the cross-sectional image data, wherein the target extraction unit applies a digitizing process, using a second threshold value, to the three-dimensional image data within the set three-dimensional region of interest.

According to another aspect of the present invention, it is preferable that, in the ultrasonic diagnostic apparatus, the two-dimensional region of interest is set surrounding a portion in which the extraction of data using the first threshold value isinaccurate. According to another aspect of the present invention, it is preferable that, in the ultrasonic diagnostic apparatus, the cross-sectional image data generation unit generates three sets of cross-sectional image data which are orthogonal to each other, and the two-dimensional region of interest is set within at least one set of cross-sectional image data from among the three sets of cross-sectional image data. According to another aspect of the present invention, it is preferable that, in the ultrasonic diagnostic apparatus, the two-dimensional region of interest is set based on a drawing operation which is performed by a user while viewing a cross-sectional image. According to another aspect of the present invention, it is preferable that, in the ultrasonic diagnostic apparatus, the two-dimensional region of interest is selected from among a plurality of prerecorded shape data.

According to another aspect of the present invention, it is preferable that, in the ultrasonic diagnostic apparatus, the three-dimensional region-of-interest setting unit generates a plurality of two-dimensional regions of interest by stepwise reducing the two-dimensional region of interest and generates the three-dimensional region of interest by superimposing the plurality of two-dimensional regions of interest with a predetermined spacing between each other. According to another aspect of the present invention, it is preferable that, in the ultrasonic diagnostic apparatus, the three-dimensional region-of-interest setting unit generates the three-dimensional region of interest by rotating the two-dimensional region of interest.

According to another aspect of the present invention, it is preferable that, in the ultrasonic diagnostic apparatus, a display image in which the target is projected onto a plane is generated using a volume rendering method based on the three-dimensional image data in which the digitizing processes are applied using the first threshold value and the second threshold value and the data corresponding to the target is extracted.

According to another aspect of the present invention, there is provided an image processing method comprising the steps of applying a digitizing process to three-dimensional image data including a target using a first threshold value to extract data corresponding to the target, generating cross-sectional image data after the digitizing process using the first threshold value from the three-dimensional image data, setting a three-dimensional region of interest within the three-dimensional image data based on a two-dimensional region of interest which is set within the cross-sectional image data, and applying a digitizing process to the three-dimensional image data using a second threshold value within the set three-dimensional region of interest.

The image processing method is executed, for example, in an ultrasonic diagnostic apparatus. Alternatively, it is also possible to execute the method by operating a computer using a program corresponding to the method.

According to another aspect of the present invention, it is preferable that, in the image processing method, a plurality of two-dimensional regions of interest are generated by stepwise shrinking the two-dimensional region of interest and the three-dimensional region of interest is generated by superimposing the plurality of two-dimensional regions of interest with a predetermined spacing between each other. According to another aspect of the present invention, it is preferable that, in the image processing method, the two-dimensional region of interest is set surrounding a portion in which extraction of data using the first threshold value is inaccurate. According to another aspect of the present invention, it is preferable that, in the image processing method, three sets of cross-sectional image data which are orthogonal to each other are generated as the cross-sectional image data and the two-dimensional region of interest is set within at least one set of cross-sectional image data from among the three sets of cross-sectional image data.

According to another aspect of the present invention, it is preferable that, in the image processing method, the two-dimensional region of interest is set based on a drawing operation which is performed by a user while the user views a cross-sectional image. According to another aspect of the present invention, it is preferable that, in the image processing method, the two-dimensional region of interest is selected from among a plurality of shape data which are recorded in advance. According to another aspect of the present invention, it is preferable that, in the image processing method, a display image in which a target is projected onto a plane is generated using a volume rendering method based on the three-dimensional image data in which the digitizing processes are applied using the first threshold value and the second threshold value and data corresponding to the target is extracted. According to another aspect of the present invention, it is preferable that, in the image processing method, the three-dimensional region of interest is generated by rotating the two-dimensional region of interest.

As described, with the present invention, it is possible to suitably identify and separate the image of a tissue or the like in a region of interest.

## BRIEF DESCRIPTION OF THE DRAWINGS

A preferred embodiment of the present invention will be described in detail based on the following figures, wherein:

Fig. 1 is a block diagram showing an overall structure of an ultrasonic diagnostic apparatus according to a preferred embodiment of the present invention;

Fig. 2 is a diagram for explaining an image displayed on a monitor;

Fig. 3 is a diagram for explaining a boundary in a debris portion;

Fig. 4 is a diagram for explaining a two-dimensional region of interest;

Fig. 5 is a diagram for explaining a process for generating a three-dimensional region of interest;

Fig. 6 is a diagram for explaining a reduction process when a three-dimensional region of interest is generated;

Fig. 7 is a diagram for explaining a reducing rate;

Fig. 8 is a diagram for explaining a reducing ratio;

Fig. 9 is a diagram for explaining a three-dimensional region of interest generated through the reduction process; and

Fig. 10 is a diagram for explaining another process for generating a three-dimensional region of interest from a two-dimensional region of interest.

## DESCRIPTION OF PREFERRED EMBODIMENT

A preferred embodiment (hereinafter referred to simply as the "embodiment") of the present invention will now be described referring to the drawings.

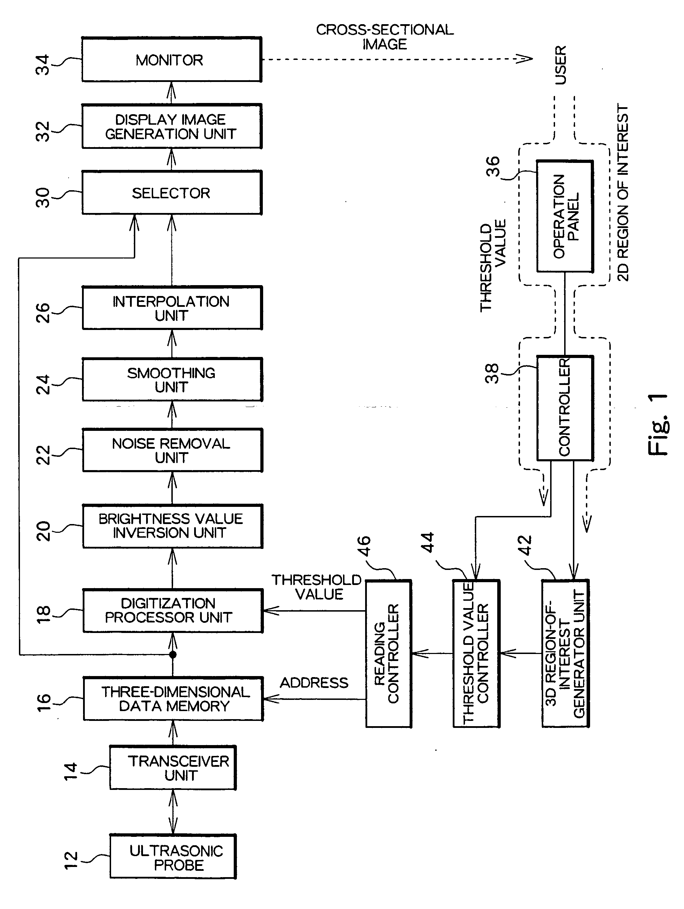

Fig. 1 is a block diagram showing an overall structure of an ultrasonic diagnostic apparatus according to the preferred embodiment of the present invention. A diagnosis target ("target volume" or simply "target") of the ultrasonic diagnostic apparatus of the present embodiment is, for example, a heart or another organ, a cavity within an organ, a liver cyst, a tumor, or the like. In the following description, the present embodiment will be described exemplifying a liver cyst as the diagnosis target.

An ultrasonic probe 12 can emit, for scanning a target, an ultrasonic beam, for example, in two directions, in order to generate a three-dimensional ultrasonic image. A transceiver unit 14 corresponds to the three-dimensional ultrasonic probe 12, controls transmission and reception of an ultrasound, and transmits received data to a three-dimensional data memory 16 which stores the data. When a convex type probe is used as the ultrasonic probe 12, the three-dimensional data in the present embodiment is stored represented in a polar coordinate system $(\theta, \varphi, r)$ with $\theta$ being a main scan direction of the ultrasonic beam, $\varphi$ being a sub-scanning direction which is perpendicular to the main scan direction, and $r$ being a distance from a center of curvature of the contact surface of the ultrasonic probe. Alternatively, the storage form of the three dimensional data may be data converted from representation in the polar coordinate system which is directly obtained from the information of the reflection wave to representation in another coordinate system. For example, the data may be stored represented in a Cartesian coordinate system $(x, y, z)$.

Data (three-dimensional image data composed of a plurality of voxel data) stored in the three-dimensional data memory 16 corresponds to a brightness corresponding to the intensity of the reflected wave. When the diagnosis target is a liver cyst, the brightness corresponding to a region outside and other than the liver cyst having a high reflection is high and the brightness corresponding to a region in the liver cyst having a low reflection is low. In consideration of this, a digitization processor unit 18 applies a digitizing process to the data in the three-dimensional data memory 16 using a predetermined threshold value. The digitizing process is first applied using a first threshold value, which may be a value preset in the apparatus or may be a value which can be set by an operator based on a viewed ultrasonic image. The first threshold value is set with the target being a region other than the debris portion of the liver cyst. More specifically, the first threshold value is set so that the liver cyst and the tissues outside the liver cyst can be suitably separated in regions other than the debris portion. For example, when the data is brightness data of 256 gradations, the first threshold value may be set at 40. Then, brightness value in each voxel is set at a low level when the brightness data corresponding to the voxel is less than the first threshold value and at a high level when the brightness data is greater than or equal to the first threshold value. In the debris portion, another digitizing process will be applied using a second threshold value, as will be described in more detail below.

A brightness value inversion unit 20 applies to the image data to which the digitizing process is applied a process of inverting the brightness value. That is, among the image data after the digitizing process is applied, the image data with a brightness value of a low level is converted to image data with a brightness value of a high level and the image data with a brightness value of a high level is converted to image data with a brightness value of a low level. As a result, the portion of the liver cyst which has a low reflection is converted a high level (high brightness), and the region outside the liver cyst which has a high reflection is converted to a low level (low brightness). The order of the digitizing and inversion processes may be reversed to obtain similar results. When the inversion process is to be applied prior to the digitizing process, for example, for data of 256 gradations, a brightness value of 0 is inverted to 256, a brightness value of 1 is inverted to 255, a brightness value of 2 is inverted to 254, ... and a brightness value of 256 is inverted to 0. Then, the digitizing process is applied using a predetermined brightness threshold value.

When the image data to which the digitizing process and the brightness value inversion process are applied is to be displayed without further processing, viewing of the displayed image is likely to be difficult because the contrast is too high and noise will have been augmented. Therefore, it is necessary to apply an image process as described below to the digitized and inverted data to generate an image which can easily be seen and clearly displayed.

A noise removal unit 22 applies a noise removal process. For example, when 5 brightness values in 8 voxels surrounding a certain voxel (noted voxel) on a $\theta$-$\varphi$ plane are at the high level, the brightness value of the noted voxel is set to the high level; when the number of voxels which are at the high level is less than 5, the original brightness value of the noted voxel is maintained; when brightness values of 5 voxels surrounding a noted voxel is at the low level, the brightness value of the noted voxel is set at the low level; and, when the number of voxels which are at the low level is less than 5, the original brightness value of the noted voxel is maintained. This process of removal of noise is executed on the $\theta$-$\varphi$ plane, but it is also possible to execute the noise removal process on the $\theta$-r plane or on the $\varphi$-r plane. In addition, it is also possible to employ a configuration in which the brightness value of a certain voxel is determined based on the brightness values of 26 surrounding voxels in a three-dimensional space.

Then, a smoothing unit 24 applies a smoothing process. As described, because the image defined by the digitized data may be unclear or confusing, an image process is applied so that the display becomes smooth. For example, by determining a brightness value of a certain voxel (noted voxel) as an average value of the brightness values of the noted voxel and surrounding voxels, it is possible to smooth the brightness values. It is possible to use 9 voxels within one plane as the target of calculation of the average or 27 voxels in three-dimensional structure. By applying the smoothing process, voxels having an intermediate gradation between the low level and the high level are created and a smooth display can be realized. Then, an interpolation unit 26 interpolates between lines ($\theta$ direction) and between frames ($\varphi$ direction).

A selector 30 selects, in response to an instruction input by the operator, one of the original three-dimensional image data stored in the three-dimensional data memory 16 or the three-dimensional image data which is digitized and inverted so that the liver cyst is extracted, and transmits the selected data to a display image generation unit 32.

A display image generation unit 32 executes a conversion process from the polar coordinates to Cartesian coordinates and an image process for a two-dimensional display. When the data stored in the three-dimensional data memory 16 is already converted to the Cartesian coordinate system as described above, the conversion in this process is a process for displaying three-dimensional data in two dimensions. As a process for displaying in two dimensions, various techniques are known such as a cross-sectional image generation of 3 orthogonal cross sections which are set within the three-dimensional image data and a volume rendering process with respect to the three-dimensional image data.

The 3 orthogonal cross sections are three cross sections which are orthogonal to each other within the data space of the three-dimensional image data and correspond to, for example, a set of a top view, a side view, and a front view. In the display image generation unit 32, voxel data on each of 3 orthogonal cross sections are extracted from the three-dimensional image data and three cross-sectional images are generated.

For the volume rendering process, it is preferable to use a method shown in, for example, Japanese Patent Laid-Open Publication No. Hei 10-33538. In this method, a viewpoint and a screen are defined sandwiching a three-dimensional data space and a plurality of rays (sight lines) are defined from the viewpoint to the screen. Then, for each ray, voxel data present on the ray are sequentially read from the three-dimensional image data and a voxel calculation (here, a calculation of an amount of output light using opacity based on the volume rendering method) is sequentially executed for each voxel data. The final result of the voxel calculation (amount of output light) is converted to a pixel value, and a two-dimensional display image in which the three-dimensional image is transmissively displayed is generated by mapping the pixel value of each ray on the screen.

The 3 orthogonal cross-sectional images or the two-dimensional display image by the volume rendering method which is generated in the display image generation unit 32 are displayed on a monitor 34.

In the following description, the present embodiment will be described with the reference numerals of Fig. 1 assigned to the sections shown on Fig. 1 and also referring to other drawings.

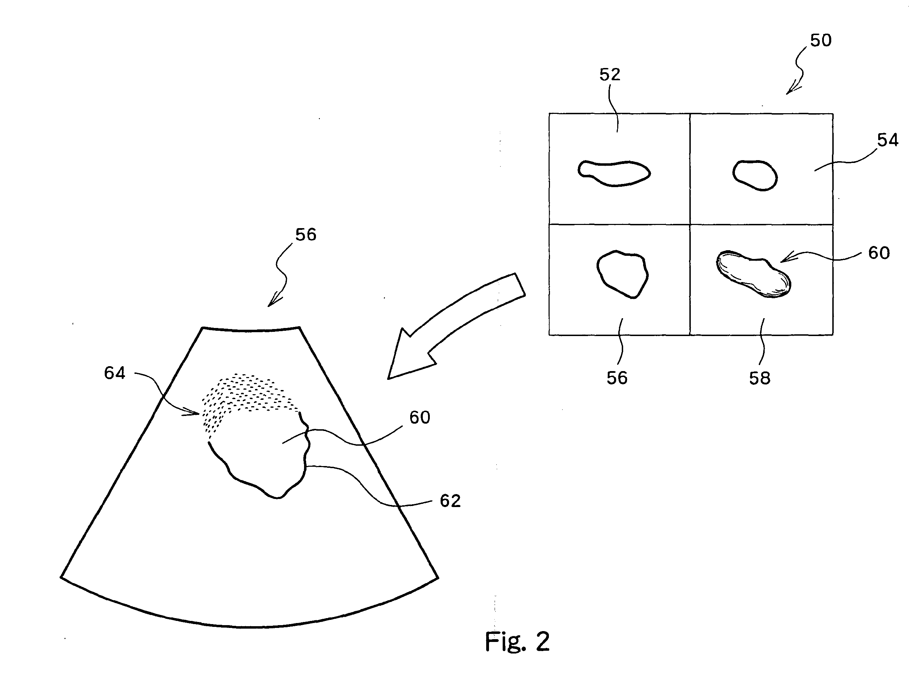

Fig. 2 is a diagram for explaining a display image 50 to be displayed on the monitor 34. The display image 50 contains 3 orthogonal cross sections (a top view 52, a side view 54, and a front view 56) and a two-dimensional display image (3D image) 58 obtained by volume rendering, regarding a liver cyst 60. As the display image 50, not all of these images are necessary. For example, it is possible to display only the 3D image 58 or display only the 3 orthogonal cross sections.

Fig. 2 also shows an enlarged view of the front view 56. As described above, debris is present in the liver cyst 60 and a boundary 62 of the liver cyst 60 is not clear at the debris portion 64 in the ultrasonic image. That is, the echo of the debris constitutes a noise and the boundary 62 between the liver cyst 60 and the tissue outside the liver cyst 60 becomes unclear. Because of this, when a digitizing process is applied in the digitization processor unit 18 using only the first threshold value, the boundary 62 at the debris portion 64 may be erroneously recognized.

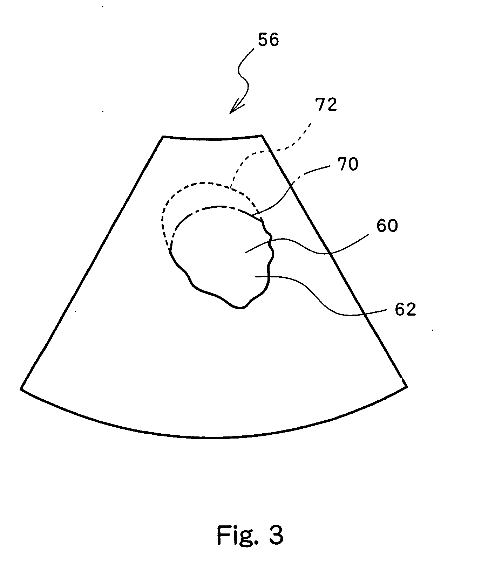

Fig. 3 is a diagram for explaining a boundary at the debris portion and shows the liver cyst 60 displayed in the front view 56. As described before, the first threshold value used in the digitization processor unit 18 is set so that the liver cyst 60 and the tissues outside the liver cyst can be suitably separated in regions other than the debris portion. Because of this, in the digitizing process using the first threshold value, the separation may be inaccurate at the debris portion and an error may occur between the boundary 70 determined using the first threshold value and the actual boundary 72.

Therefore, in the present embodiment, a second threshold value, which, although it may assume the same value as the first threshold value, is a separate value from the first threshold value (is set in the debris portion and a special digitizing process using the second threshold value is applied only to the debris portion. In this process, a two-dimensional region of interest for specifying the debris portion is set, a three-dimensional region of interest is generated from the two-dimensional region of interest, and a digitizing process with respect to the three-dimensional image data is executed based on the three-dimensional region of interest.

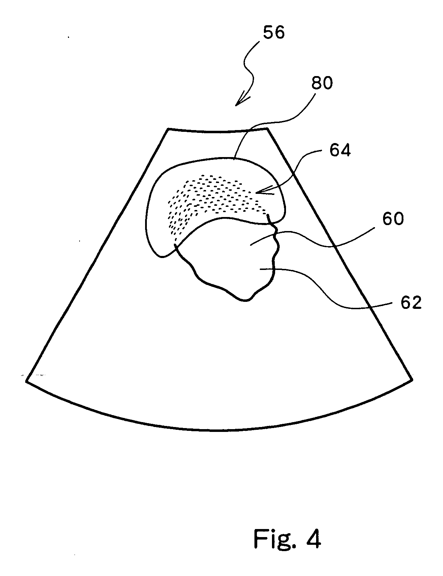

Fig. 4 is a diagram for explaining a two-dimensional region of interest and shows a two-dimensional region 80 of interest which is set in the front view 56. The two-dimensional region 80 of interest is set so as to surround the debris portion 64 of the liver cyst 60. The two-dimensional region 80 of interest is set, for example, based on a drawing operation by an operator (user) of the ultrasonic diagnostic apparatus using an operation panel 36 while viewing an image displayed on the monitor 34. Alternatively, the two-dimensional region 80 of interest may be selected from among a plurality of shape data which are recorded in the apparatus in advance. The two-dimensional region 80 of interest may alternatively be set within the top view or side view (refer to Fig. 2).

In the present embodiment, a three-dimensional region of interest is generated from the set two-dimensional region 80 of interest. The three-dimensional region of interest is generated by a 3D region-of-interest generator unit 42. Specifically, the 3D region-of-interest generator unit 42 generates a three-dimensional region of interest based on the setting information of the two-dimensional region 80 of interest transmitted via a controller 38.

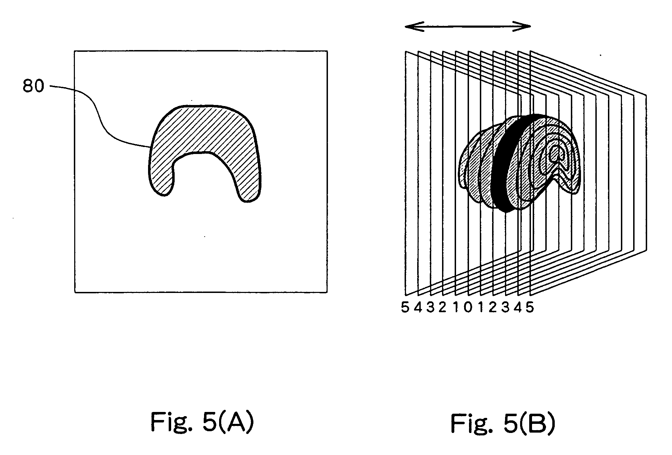

Fig. 5 is a diagram for explaining a process of generating the three-dimensional region of interest. The 3D region-of-interest generator unit 42 generates a plurality of two-dimensional regions 80 of interest by stepwise reduction of the two-dimensional region 80 of interest and the plurality of two-dimensional regions 80 of interest are superimposed with a predetermined spacing to each other to generate a three-dimensional region of interest. In other words, with the two-dimensional region 80 of interest shown in (A) as a basis, the base two-dimensional region 80 of interest is placed at a position of "0" in (B) and the two-dimensional region 80 of interest are placed in positions of "1", "2", "3", "4", and "5" shown in (B) while the base two-dimensional region 80 of interest is reduced in steps, so that a three-dimensional region of interest is generated as a collection of a plurality of two-dimensional regions 80 of interest.

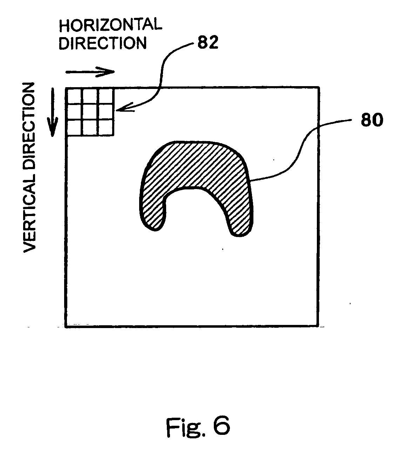

Fig. 6 is a diagram for explaining a reduction process when the three-dimensional region of interest is generated. The 3D region-of-interest generator unit 42 provides a 3x3 window 82, that is, a window 82 having 3 pixels in a horizontal direction and 3 pixels in a vertical direction with a total of 9 pixels, within a plane in which the two-dimensional region of interest 80 is set. The plane in which the two-dimensional region 80 of interest is set is scanned with the 3x3 window 82 in the horizontal and vertical directions, and a reduction process is achieved by determining a center pixel value from 9 pixel values within the window 82 in each scanned position. More specifically, when the pixel value of the pixels in the two-dimensional region 80 of interest is H and the pixel values of the other pixels is L, for example, when there is at least one pixel having an L in the 9 pixels of the window 82, the center pixel of the window 82 is replaced with L. One round of reduction processing is completed by performing this replacement process while the entire region of the plane is scanned with the window 82. After one round of the reduction process, the two-dimensional region 80 of interest is reduced by approximately one pixel in the periphery.

In addition, by applying the conversion process of the pixel value over the entire plane while the two-dimensional region 80 of interest obtained by one round of the reduction process is scanned with the window 82 in the horizontal and vertical directions, a second round of reduction processing is executed. After two rounds of reduction processing, the two-dimensional region 80 of interest is reduced in size by an amount corresponding to two pixels in the periphery compared to the base image. A third round, a fourth round, ... of reduction processing can be similarly applied.

A tissue within a living body can be considered as basically having an ellipsoidal shape which is round. Therefore, the 3D region-of-interest generator unit 42 generates the three-dimensional region of interest so that the region is ellipsoidal. For example, when the two-dimensional region of interest is circular, a three-dimensional region of interest having a spherical shape is generated, and, when the two-dimensional region of interest is crescent-shaped, a three-dimensional region of interest in the shape of a banana is generated. For this purpose, the 3D region-of-interest generator unit 42 stepwise proceeds with the reduction process by a predetermined reducing rate and a predetermined reducing ratio (reducing value).

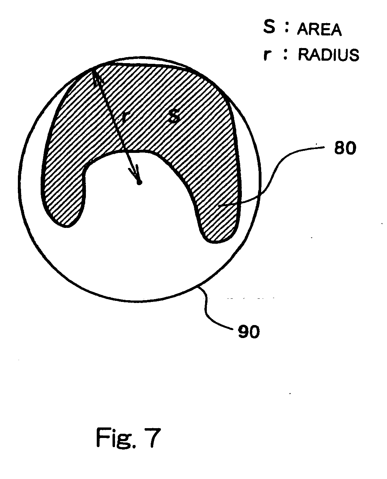

Fig. 7 is a diagram for explaining a reducing rate. The reducing rate R is defined by the following equation 1:

[Equation 1]

$$R = \pi\, r^2/S$$

wherein an area S represents an area of the two-dimensional region 80 of interest which is the basis before the reduction process and a radius r represents a radius of a circumscribing circle 90 of the two-dimensional region of interest. The reducing rate R defined by the equation 1 corresponds to a distance between the plurality of two-dimensional regions of interest generated as a result of the reduction process, that is, a distance between a plurality of planes shown in Fig. 5 (B).

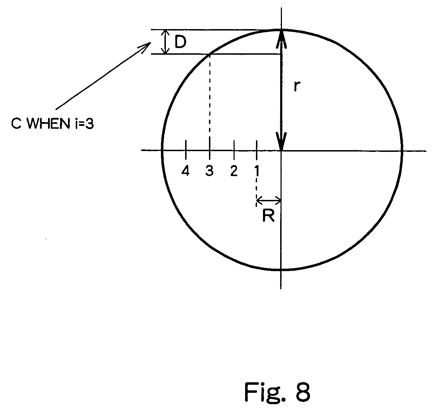

Fig. 8 is a diagram for explaining a reducing ratio. The reducing ratio C is defined by the following equation 2:

[Equation 2]

$$C = r - \sqrt{r^2 - (R \times i)^2}$$

wherein the reducing rate R is determined from equation 1, a radius r represents a radius of a circumscribing circle (represented by reference numeral 90 in Fig. 7) of the two-dimensional region of interest, and i represents a plane number and corresponds to a position of the plane such as "0", "1", "2", "3", "4", and "5" in Fig. 5 (B). For example, when the plane number is 3 (i = 3), the reducing ratio C obtained from the equation 2 corresponds to the distance D shown in Fig. 8.

The 3D region-of-interest generator unit 42 executes reduction processing with a reducing value corresponding to the reducing ratio C. That is, a reducing ratio C is calculated using the equation 2 for each plane number i, and a two-dimensional region of interest corresponding to each plane number is generated through reduction processes repeated for a number of rounds corresponding to the reducing ratio C. For example, for a plane at the position "1" shown in Fig. 5 (B), C rounds of reduction processes (corresponding to reduction of approximately C pixels at the periphery) are performed based on the reducing ratio C obtained from i = 1 to generate a two-dimensional region of interest. Similarly, regarding a plane at the position "2" shown in Fig. 5 (B), a two-dimensional region of interest is generated through C rounds of reduction processing based on the reducing ratio C obtained from i = 2. As a result, two-dimensional regions of interest to which the reduction processes are applied are superimposed, gradually becoming rounder.

When the value of C obtained in the equation 2 is non-integer, it is possible to use a maximum integer less than C as the reducing value. A maximum value n of i is n = r/R, and this value n corresponds to the number of planes superimposed toward one direction from the position "0" shown in Fig. 5 (B).

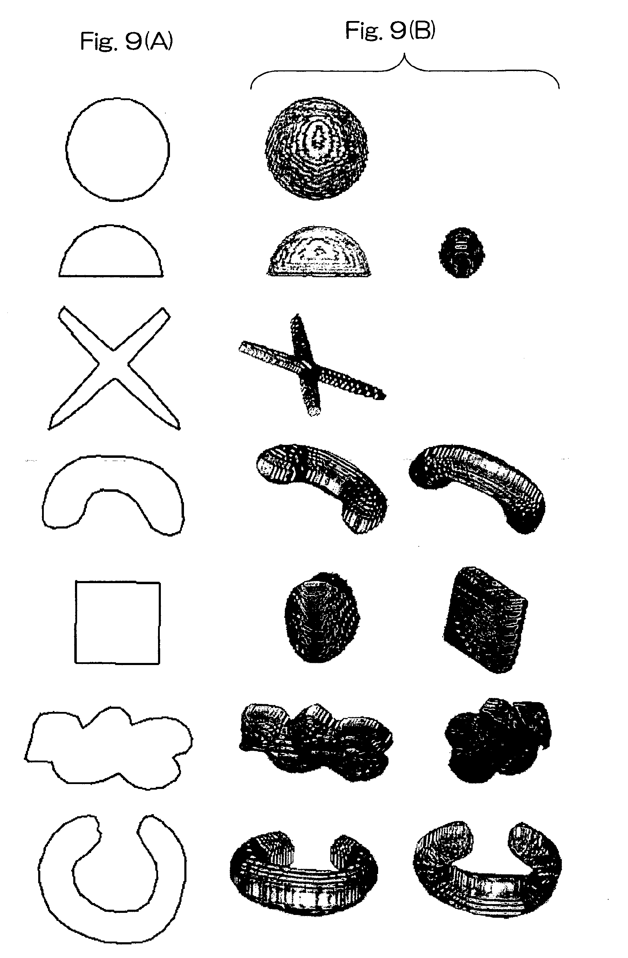

Fig. 9 is a diagram for explaining a three-dimensional region of interest generated in the reduction process explained referring to Figs. 5 - 8. Fig. 9 (A) shows a base two-dimensional region of interest and Fig. 9 (B) shows a three-dimensional region of interest obtained from the two-dimensional regions of interest. As shown in Fig. 9, basically, the three-dimensional region of interest has a shape in which the corresponding two-dimensional region of interest is expanded in a rounder shape.

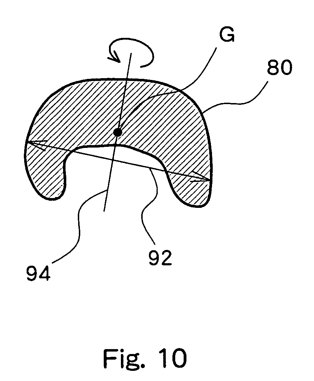

Fig. 10 is a diagram for explaining another process for generating a three-dimensional region of interest from a two-dimensional region of interest. The 3D region-of-interest generator unit 42 generates a three-dimensional region of interest by rotating a two-dimensional region 80 of interest about a center line 94 of the two-dimensional region of interest. In other words, a normal of a line segment 92 which passes through a center of gravity G of the two-dimensional region 80 of interest and connecting two most-distanced points within the two-dimensional region 80 of interest is used as the center line 94 and the two-dimensional region 80 of interest is rotated about the center line 94 to generate the three-dimensional region of interest.

The 3D region-of-interest generator unit 42 generates a three-dimensional region of interest from a two-dimensional region of interest through a shrinking process explained referring to Figs. 5 - 8 or through the rotation process explained referring to Fig. 10.

Returning to Fig. 1, when the three-dimensional region of interest is generated, a threshold value controller 44 sets a second threshold value to be used in the generated three-dimensional region of interest. For example, the user inputs a suitable value from the operation panel 36 while viewing a cross-sectional image displayed on the monitor 34, the input value is transmitted to the threshold value controller 44 via the controller 38, and the second threshold value is set based on the input value.

When the second threshold value is set, a reading controller 46 controls the threshold value used in the digitization processor unit 18 based on an address within the three-dimensional data memory 16. In other words, when image data present in the three-dimensional region of interest is read, a digitizing process is applied using the second threshold value, and, when other image data is read, a digitizing process is applied using the first threshold value. Therefore, for example, in Fig. 3, the boundary 62 is extracted using the first threshold value in regions other than the debris portion and the second threshold value is suitably set in the debris portion to extract the actual boundary 72.

A preferred embodiment of the present invention has been described. However, the above-described embodiment is only exemplary and should not be construed to be limiting the scope of the present invention. For example, although in the embodiment a single three-dimensional region of interest is set, it is also possible to set a plurality of three-dimensional regions of interest. In such case, a threshold value is set for each three-dimensional region of interest, and, for example, three or more threshold values such as a third threshold value and a fourth threshold value may be set.

**Claims**

1. An ultrasonic diagnostic apparatus comprising:

   an image data generation unit which transmits and receives an ultrasound to and from a space including a target to generate ultrasonic image data;
   a target extraction unit which applies a digitizing process to the ultrasonic image data using a first threshold value to extract data corresponding to the target; and
   a region-of-interest setting unit which sets a region of interest within the ultrasonic image data, wherein
   the target extraction unit applies a digitizing process, using a second threshold value, to the ultrasonic image data within the set region of interest.

2. An ultrasonic diagnostic apparatus according to Claim 1, wherein the region-of-interest setting unit sets a region of interest surrounding a portion within the ultrasonic image data in which the extraction of data using the first threshold value is inaccurate.

3. An ultrasonic diagnostic apparatus comprising:

   a three-dimensional image data generation unit which transmits and receives an ultrasound to and from a space including a target to generate three-dimensional image data;
   a target extraction unit which applies a digitizing process to the three-dimensional image data using a first threshold value to extract data corresponding to the target;
   a cross-sectional image data generation unit which generates, from the three-dimensional image data, cross-sectional image data after application of the digitizing process using the first threshold value; and
   a three-dimensional region-of-interest setting unit which sets a three-dimensional region of interest within the three-dimensional image data based on a two-dimensional region of interest which is set within the cross-sectional image data, wherein
   the target extraction unit applies a digitizing process, using a second threshold value, to the three-dimensional image data within the set three-dimensional region of interest.

4. An ultrasonic diagnostic apparatus according to Claim 3, wherein
   the two-dimensional region of interest is set surrounding a portion in which the extraction of data using the first threshold value is inaccurate.

5. An ultrasonic diagnostic apparatus according to Claim 4, wherein
   the cross-sectional image data generation unit generates three sets of cross-sectional image data which are orthogonal to each other, and
   the two-dimensional region of interest is set within at least one set of cross-sectional image data from among the three sets of cross-sectional image data.

6. An ultrasonic diagnostic apparatus according to Claim 5, wherein
   the two-dimensional region of interest is set based on a drawing operation which is performed by a user while viewing a cross-sectional image.

7. An ultrasonic diagnostic apparatus according to Claim 5, wherein
   the two-dimensional region of interest is selected from among a plurality of shape data which are recorded in advance.

8. An ultrasonic diagnostic apparatus according to Claim 4, wherein
   the three-dimensional region-of-interest setting unit generates a plurality of two-dimensional regions of interest by stepwise reduction of the two-dimensional region of interest and generates the three-dimensional region of interest by superimposing the plurality of two-dimensional regions of interest with a predetermined spacing between each other.

9. An ultrasonic diagnostic apparatus according to Claim 4, wherein
   the three-dimensional region-of-interest setting unit generates the three-dimensional region of interest by rotating the two-dimensional region of interest.

10. An ultrasonic diagnostic apparatus according to Claim 4, wherein
    a display image in which the target is projected onto a plane is generated using a volume rendering method based on the three-dimensional image data in which the digitizing processes are applied using the first threshold value and the second threshold value and the data corresponding to the target is extracted.

**11.** An image processing method comprising the steps of:

applying a digitizing process to three-dimensional image data including a target using a first threshold value to extract data corresponding to the target;
generating cross-sectional image data after the digitizing process using the first threshold value from the three-dimensional image data;
setting a three-dimensional region of interest within the three-dimensional image data based on a two-dimensional region of interest which is set within the cross-sectional image data; and
applying a digitizing process to the three-dimensional image using a second threshold value within the set three-dimensional region of interest.

**12.** An image processing method according to Claim 11, wherein
a plurality of two-dimensional regions of interest are generated by stepwise reduction of the two-dimensional region of interest and the three-dimensional region of interest is generated by superimposing the plurality of two-dimensional regions of interest with a predetermined spacing between each other.

**13.** An image processing method according to Claim 12, wherein
the two-dimensional region of interest is set surrounding a portion in which the extraction of data using the first threshold value is inaccurate.

**14.** An image processing method according to Claim 13, wherein
three sets of cross-sectional image data which are orthogonal to each other are generated as the cross-sectional image data, and
the two-dimensional region of interest is set within at least one set cross-sectional image data from among the three sets of cross-sectional image data.

**15.** An image processing method according to Claim 14, wherein
the two-dimensional region of interest is set based on a drawing operation which is performed by a user while viewing a cross-sectional image.

**16.** An image processing method according to Claim 14, wherein
the two-dimensional region of interest is selected from among a plurality of shape data which are recorded in advance.

**17.** An image processing method according to Claim 14, wherein
a display image in which the target is projected onto a plane is generated using a volume rendering method based on the three-dimensional image data in which the digitizing processes are applied using the first threshold value and the second threshold value and data corresponding to the target is extracted.

**18.** An image processing method according to Claim 11, wherein
the three-dimensional region of interest is generated by rotating the two-dimensional region of interest.

Fig. 1

Fig. 2

11

Fig. 3

Fig. 4

80

5 4 3 2 1 0 1 2 3 4 5

Fig. 5(A)     Fig. 5(B)

HORIZONTAL
DIRECTION 82

VERTICAL DIRECTION

80

Fig. 6

Fig. 7

C WHEN i=3

Fig. 8

EP 1 600 891 A1

Fig. 9(A)

Fig. 9(B)

Fig. 10

**European Patent Office**

**EUROPEAN SEARCH REPORT**

Application Number

EP 05 00 9137

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2003/174890 A1 (YAMAUCHI MASAKI) 18 September 2003 (2003-09-18) | 1,2 | G06T7/00 A61B8/14 A61B8/08 G01S7/52 |
| A | * First Embodiment * ----- | 3-18 | |
| X | US 2002/102023 A1 (YAMAUCHI MASAKI) 1 August 2002 (2002-08-01) | 1,2 | |
| A | * paragraph [0124] * ----- | 3-18 | |

TECHNICAL FIELDS SEARCHED (Int.Cl.7)

A61B
G06T
G01S

The present search report has been drawn up for all claims

| Place of search | Date of completion of the search | Examiner |
|---|---|---|
| Berlin | 29 August 2005 | Clevorn, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 05 00 9137

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2005

| Patent document cited in search report | | Publication date | Patent family member(s) | | Publication date |
|---|---|---|---|---|---|
| US 2003174890 | A1 | 18-09-2003 | CA<br>CN<br>EP<br>JP | 2421468 A1<br>1444907 A<br>1400920 A2<br>2003334194 A | 14-09-2003<br>01-10-2003<br>24-03-2004<br>25-11-2003 |
| US 2002102023 | A1 | 01-08-2002 | JP | 2002224116 A | 13-08-2002 |

EPO FORM P0459

For more details about this annex : see Official Journal of the European Patent Office, No. 12/82